# EUROPEAN PATENT APPLICATION

(11) **EP 3 323 432 A2**
(43) Date of publication of application: **23.05.2018**
(21) Application number: 17201929.1
(22) Date of filing: 15.11.2017
(51) Int. Cl.: A61L 2/06, B65B 53/06

(54) **STERILIZATION AND DEPYROGENATION MACHINE**

(30) Priority: 16.11.2016 IT 201600115684
(71) Applicant: Boldrini, Massimo, 53035 Monteriggioni, SI (IT)
(72) Inventor: Boldrini, Massimo, 53035 Monteriggioni, SI (IT)
(74) Representative: Modiano, Micaela Nadia

(57) **Abstract**

A sterilization and depyrogenation machine (1) for containers for chemical-pharmaceutical use, particularly of the energy-saving type, comprising a plurality of thermal chambers (2, 3, 4) adapted to perform a process for the sterilization and depyrogenation of objects (5) for chemical-pharmaceutical use and arranged mutually in sequence along an advancement line (6) of the objects (5) in a thermally independent manner,
the thermal chambers (2, 3, 4) being provided with openings for the entry and exit of the objects (5) which are formed transversely along and with respect to the advancement line (6) in such a manner as to give the machine (1) a tunnel-like configuration,
the machine further comprising means (14) for the advancement of the objects (5) which are adapted to transfer them through the thermal chambers (2, 3, 4) and are arranged mutually in sequence along the advancement line (6) so as to define a plurality of advancement sectors (15, 16, 17), which are equal in number to at least one for each thermal chamber (2, 3, 4), so that each one of the advancement means (14) that belongs to the same advancement sector (15, 16, 17) undergoes the thermal influence of only one thermal chamber (2, 3, 4).

## Description

The present invention relates to a sterilization and depyrogenation machine for containers for chemical-pharmaceutical use, particularly of the energy-saving type.

In the chemical-pharmaceutical industry, it is known to perform sterilization and depyrogenation processes on containers, such as ampoules and/or bottles made of glass, aluminum or stainless steel, adapted to contain sterile and non-pyrogenic drugs.

These processes provide for a thermal treatment that essentially consists in raising the temperature of these containers to a maximum of 350°C with dry heat and with subsequent cooling to ambient temperature or higher at no more than 10°C.

This thermal treatment can occur intermittently by means of static stoves (maximum temperature = 250°C) or in a continuous manner in sterilization and depyrogenation machines configured like a tunnel, in which a laminar air flow is generated (maximum temperature = 350°C).

The use of these sterilization and depyrogenation machines for medium and large productions is well-established and the constructive design method is similar for European and American manufacturers that are present in the current market.

In most cases, this tunnel-like machine is inserted downstream of a washing machine for containers, which operates in a sterile environment, and upstream of machines for the dosage and closure of said containers, which operate in a contaminated environment.

Sterilization and depyrogenation machines of the known type are formed by three contiguous thermal chambers crossed by a conveyor belt made of stainless steel, the function of which is to transfer the containers that arrive from the washing machine through the first chamber, termed entry chamber, through the second chamber, termed depyrogenation chamber, and through the third chamber, termed cooling chamber.

In greater detail, the entry chamber has the function of isolating, with an overpressure of sterile filtered air, the containers that arrive from the washing machine from environmental particle contaminations and can be preheated by using the air expelled to cool the treated objects.

Dust, which as is known is the greatest vehicle of spores, bacteria and other contaminating particles, can in fact become attached to the containers that arrive from the washing machine, since they are still wet.

The depyrogenation and cooling chambers instead have the function respectively of raising the temperature of the containers to a maximum of 350°C and then of cooling them to complete the sterilization cycle and allow the execution of the steps for filling and closing said containers.

In sterilization and depyrogenation machines of the known type, movement of the containers occurs by means of a single conveyor belt, made of stainless steel in order to ideally meet the sterilization requirements, with continuous motion through the three thermal chambers, undergoing a thermal cycle that consists in:
- heating to approximately 98% of the temperature reached by the air,
- cooling to ambient temperature.

Obviously, this cycle is repeated at each full turn of the conveyor belt.

In order to limit energy dispersion in compliance with the geometries and volumes of the machines, the current trend of manufacturers is to make the conveyor belt pass in the three thermal chambers only in the container transport step, while for the return of the belt a passage that is external to the chambers is provided, thus avoiding a double heating/cooling for each complete turn.

In some cases, instead, the conveyor belt passes through the three thermal chambers in both directions, consequently increasing the energy dispersion caused by a double heating/cooling for each complete turn.

However, regardless of the solution chosen among the two that have just been presented, sterilization and depyrogenation machines of the known type are not free from drawbacks, which include the fact that they have a high energy consumption caused at least for 40%/50% by the fact that part of the energy required for the thermal treatment of the containers is wasted in order to vary continuously the temperature of the conveyor belt, which at each cycle undergoes an unnecessary heating and cooling.

Imagining a portion of the conveyor belt returning from the third thermal chamber, said portion in fact carries containers which must be heated but has a lower temperature than said containers and therefore, requiring a longer travel time, especially in the second chamber, in order to ensure that the entire container is heated according to the technical specifications of the sterilization and depyrogenation process, comprising the resting base thereof, which, being in contact with the conveyor belt, is the last part of the container to reach the desired temperature.

Another severe drawback of sterilization and depyrogenation machines of the known type resides in that if hazardous packagings, bearing for example antibiotic powders, cytotoxic substances, etc., are provided downstream of said machines, there is the risk that the conveyor belt may come into contact with these substances, transferring them to the entry area of the machine, with consequent environmental contamination.

The aim of the present invention is to provide a sterilization and depyrogenation machine for containers for chemical-pharmaceutical use that obviates the drawbacks noted above and is such as to overcome the limitations of the background art.

Within this aim, an object of the present invention is to provide a sterilization and depyrogenation machine for containers for chemical-pharmaceutical use that is capable of eliminating, in the operating condition, the energy consumption for heating and cooling caused by the system for the conveyance of the containers to be treated, allowing an energy saving comprised between 40% and 50% of the power required for the process for sterilization and depyrogenation of the treated containers.

Another object of the present invention is to provide a sterilization and depyrogenation machine for containers for chemical-pharmaceutical use that prevents the conveyance of unwanted contaminations through the system for the conveyance of the containers to be treated.

A further object of the present invention is to provide a sterilization and depyrogenation machine for containers for chemical-pharmaceutical use that offers the greatest assurances of reliability and operation.

A still further object of the present invention is to provide a sterilization and depyrogenation machine for containers for chemical-pharmaceutical use that uses production and operation technologies that are easily commercially available and is therefore easy and quick to provide and therefore economically competitive if compared with similar objects of the known type.

This aim, and these and other objects which will become better apparent hereinafter, are achieved by a sterilization and depyrogenation machine for containers for chemical-pharmaceutical use, particularly of the energy-saving type, comprising a plurality of thermal chambers adapted to perform a process for the sterilization and depyrogenation of objects for chemical-pharmaceutical use and arranged mutually in sequence along an advancement line of said objects in a thermally independent manner, said thermal chambers being provided with openings for the entry and exit of said objects which are formed transversely along and with respect to said advancement line in such a manner as to give said machine a tunnel-like configuration, characterized in that it comprises means for the advancement of said objects which are adapted to transfer said objects through said thermal chambers and are arranged mutually in sequence along said advancement line so as to define a plurality of advancement sectors, which are equal in number to at least one for each one of said thermal chambers, so that each one of said advancement means that belongs to the same one of said advancement sectors undergoes the thermal influence of only one of said thermal chambers.

Further characteristics and advantages of the invention will become better apparent from the description of a preferred but not exclusive embodiment of a sterilization and depyrogenation machine for containers for chemical-pharmaceutical use, particularly of the energy-saving type according to the invention, illustrated by way of nonlimiting example in the accompanying figures, wherein:
Figure 1 is a first perspective view of the sterilization and depyrogenation machine for containers for chemical-pharmaceutical use, particularly of the energy-saving type, according to the invention, taken from the front;
Figure 2 is a second perspective view of the machine shown in Figure 1, taken from the rear;
Figure 3 is a partially sectional lateral elevation view of the machine shown in the preceding figures;
Figure 4 is a partially exploded third perspective view of the machine shown in the preceding figures;
Figure 5 is a first perspective view of a first detail of the machine shown in the preceding figures, taken from above;
Figure 6 is a second perspective view of the detail shown in Figure 5, taken from below;
Figure 7 is a first lateral elevation view of the detail shown in Figures 5 and 6;
Figure 8 is a second lateral elevation view of the detail shown in Figures 5 to 7;
Figures 9 to 11 are three perspective views of a second detail of the machine shown in the preceding figures, showing in sequence its operation.

With particular reference to the cited figures, the sterilization and depyrogenation machine for containers for chemical-pharmaceutical use, particularly of the energy-saving type, designated generally by the reference numeral 1 in the accompanying figures, comprises a plurality of thermal chambers 2, 3 and 4 adapted for the execution of a process for the sterilization and depyrogenation of objects 5 for chemical-pharmaceutical use, such as for example containers of the type of ampoules and/or bottles made of glass, aluminum or stainless steel, adapted to contain sterile and non-pyrogenic drugs, and arranged in sequence with respect to each other along a line 6 for the advancement of the objects 5 in a thermally independent manner.

In greater detail, said thermal chambers 2, 3 and 4 are provided with entry and exit openings for the objects 5 which are formed transversely along and with respect to the advancement line 6 in such a manner as to give the machine 1 a tunnel-like configuration.

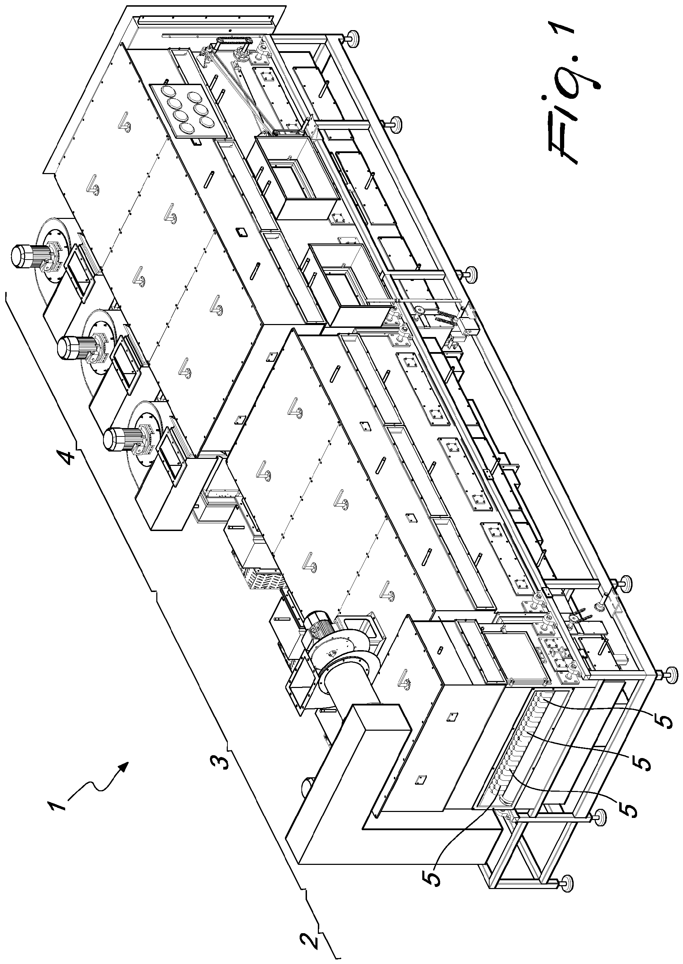

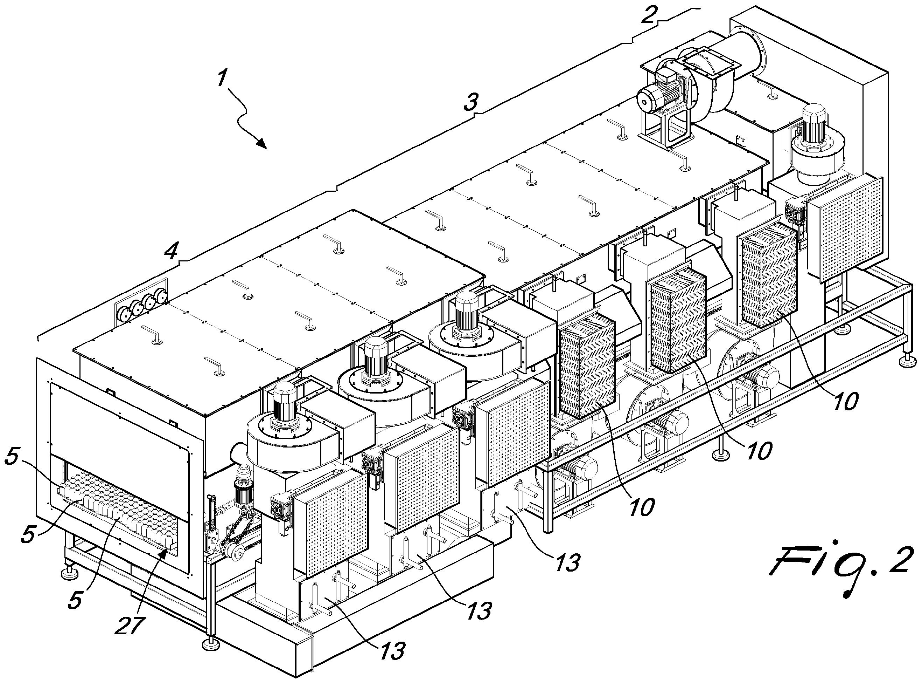

With particular reference to Figures 1 and 2, in the proposed embodiment the machine 1 described herein is configured substantially according to the traditional layout of sterilization and depyrogenation machines for containers for chemical-pharmaceutical use.

The machine 1 in fact comprises a first thermal chamber 2, termed entry chamber, which has the task of insulating, with a laminar flow 7 of sterile filtered air with absolute filters 7a in overpressure, the objects 5 that arrive from washing machines against environmental particle contamination and can be preheated by using the air expelled to cool the treated objects 5.

This is important because, as already mentioned, dust can cling to the objects 5 that arrive from washing machines, since they are still wet, and as is known is the greatest vehicle of spores, bacteria and other contaminating particles.

Subsequently there is a second thermal chamber 3, termed depyrogenation chamber, which has the function of heating the objects 5 up to their complete sterilization by means of a laminar flow 8 of air filtered with absolute filters 9 and heated with electric resistance heaters 10, which by striking vertically from above downward the objects 5 progressively transfers heat to them.

Finally, there is a third and final thermal chamber 4, termed cooling chamber, which has the task of cooling the sterilized objects 5 that exit from the second chamber 3 by means of a laminar flow 11 of air filtered with absolute filters 12 which can be cooled with refrigeration units, with electric refrigeration systems or with air/water exchangers 13 or with the intake of treated environmental air.

In this manner, the objects 5 are ready and suitable for the subsequent steps for filling and closing them.

According to the invention, there are means 14 for the advancement of the objects 5 which are adapted for the transfer of the latter through the thermal chambers 2, 3 and 4 and are arranged in sequence with respect to each other along the advancement line 6 in such a manner as to define a plurality of advancement sectors 15, 16 and 17 which are equal in number to at least one for each thermal chamber 2, 3 and 4, so that each advancement means 14 that is part of the same advancement sector 15, 16 or 17 undergoes the thermal influence of a single thermal chamber 2, 3 or 4.

Advantageously, said advancement means 14 comprise a plurality of conveyor belts 18, 19 and 20, one for each advancement sector 15, 16 and 17, which have the function of transferring sequentially the objects 5 that arrive for example from the washing machine through the thermal chambers 2, 3 and 4.

Conveniently, each one of the conveyor belts 18, 19 and 20, which in the proposed embodiment are three and are made of stainless steel and of the type that is motorized in a mutually independent and synchronous manner, forms a horizontal advancement plane that is substantially coplanar to the horizontal advancement plane defined by the consecutive conveyor belt 18, 19 or 20.

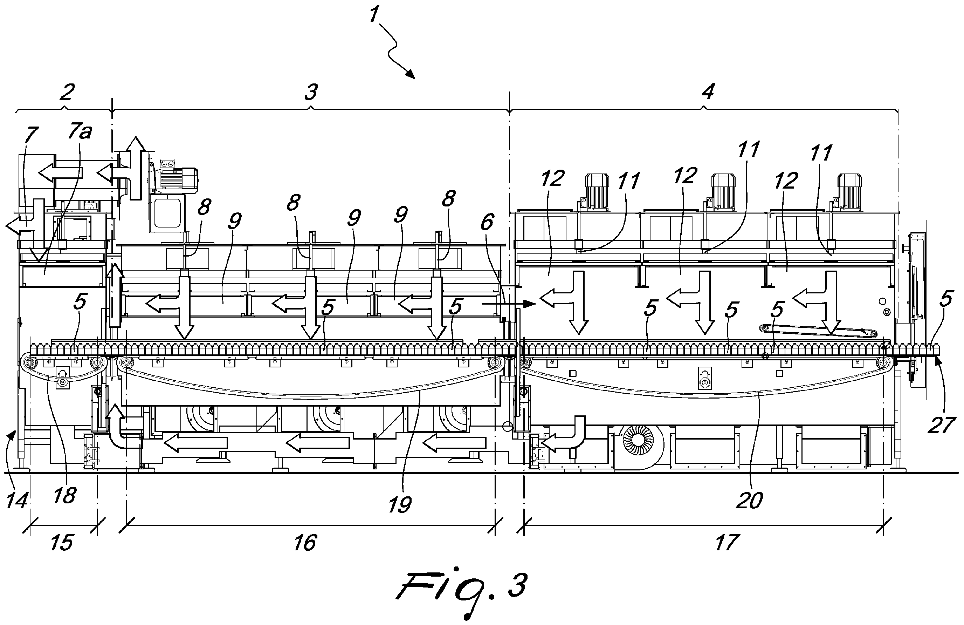

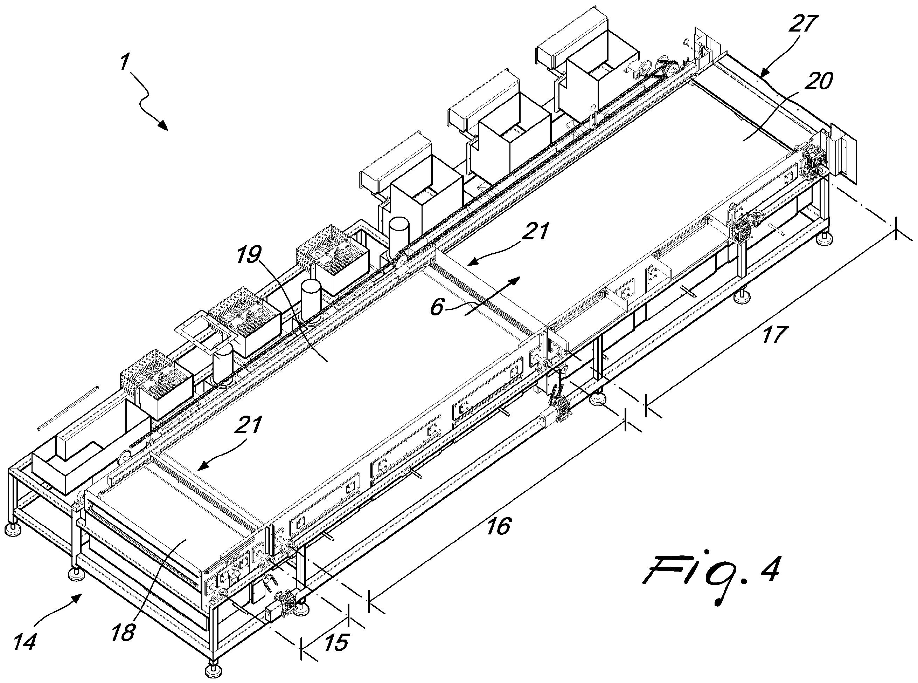

With particular reference to Figures 3 and 4, in the proposed embodiment the conveyor belts 18, 19 and 20 have a continuous motion and are fully accommodated inside the respective thermal chamber 2, 3 or 4 in such a manner as to keep its temperature substantially constant with the machine 1 in the operating condition.

Equally advantageously, with particular reference to Figures 4 to 6, there are coplanar connection means 21 formed between each pair of conveyor belts 18, 19 and 20 and adapted to allow the sliding passage of the objects 5 from each conveyor belt 18, 19 and 20 to the next one.

In greater detail, for each pair of conveyor belts 18, 19 and 20, said coplanar connection means 21 comprise at least two planar elements 22 and 23 which are interposed in sequence with respect to each other between the conveyor belts 18, 19 and 20 and are comb-shaped, so as to be mutually associated so that the teeth of one are accommodated with play in the recesses of the other.

More specifically, each planar element 22 and 23 is partially superimposed on the adjacent conveyor belt 18, 19 or 20 at the end part of the latter with an edge 24 that is rounded on the opposite side with respect to the one designed to support the objects 5 in such a manner as to support them as soon as the conveyor belt 18, 19 or 20 winds around its respective supporting roller.

Conveniently, said planar elements 22 and 23 are slidingly mutually associated along the advancement line 6 so as to compensate the thermal expansion of the material of which they are made, which can be, for example, stainless steel.

Furthermore, advantageously, the first one of each pair of said planar elements 22 and 23, established along the direction of advancement of the advancement line 6, defines a sliding plane that is parallel and is arranged at a level that is higher than or equal to that of the sliding surface defined by the second of said planar elements 22 and 23, always established along the advancement direction of the advancement line 6.

In this manner, the teeth of the second planar element 23 are prevented from acting as a hindrance to the sliding of the objects 5 that arrive from the first planar element 22.

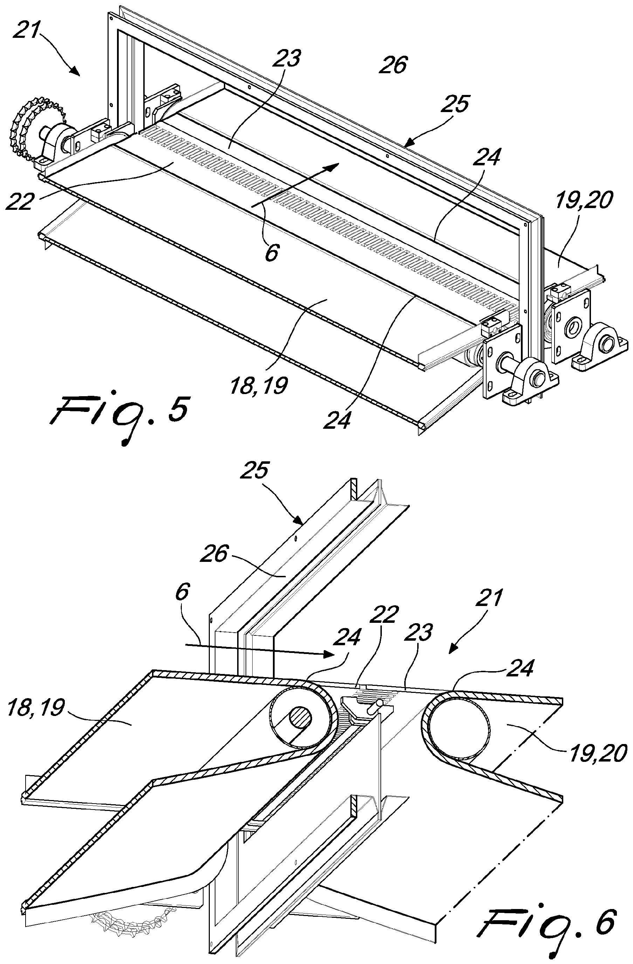

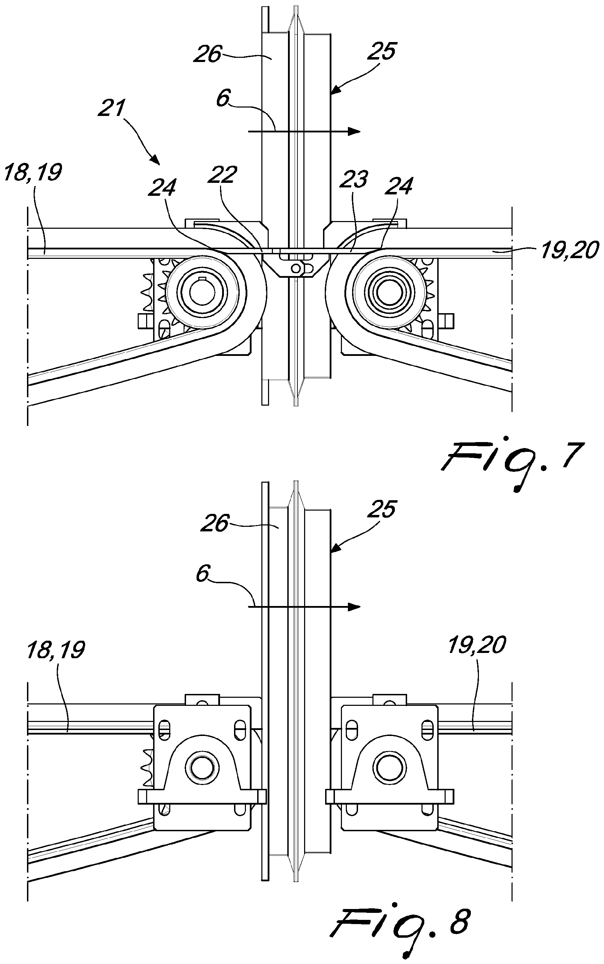

Furthermore, with particular reference to Figures 6 to 8, for each pair of said thermal chambers 2, 3 and 4 there are means 25 for compensating the thermal expansion of the hottest of said two thermal chambers 2, 3 or 4 toward the coldest of said two thermal chambers 2, 3 or 4 in such a manner as to compensate the thermal expansion of the material of which they are made, ensuring at the same time the insulation of the advancement line 6 from the outside environment both for the portions formed inside the thermal chambers 2, 3 and 4 and for the transition portions formed between one thermal chamber 2, 3 or 4 and the other.

In greater detail, in the proposed embodiment, said compensation means 25 comprise accordion structures 26 formed between the openings of two consecutive thermal chambers 2, 3 or 4.

To complete the machine 1, there is an unloading plane 27 that is arranged at the end of the last conveyor belt 20 along the advancement direction of the advancement line 6.

Conveniently, said unloading plane 27, which can be of the type with suction in partial vacuum, is partially superimposed on said last conveyor belt 20 at the end part of the latter, with an edge that is rounded on the opposite side with respect to the one designed to support the objects 5 so as to support them as soon as the conveyor belt 20 winds around its respective supporting roller.

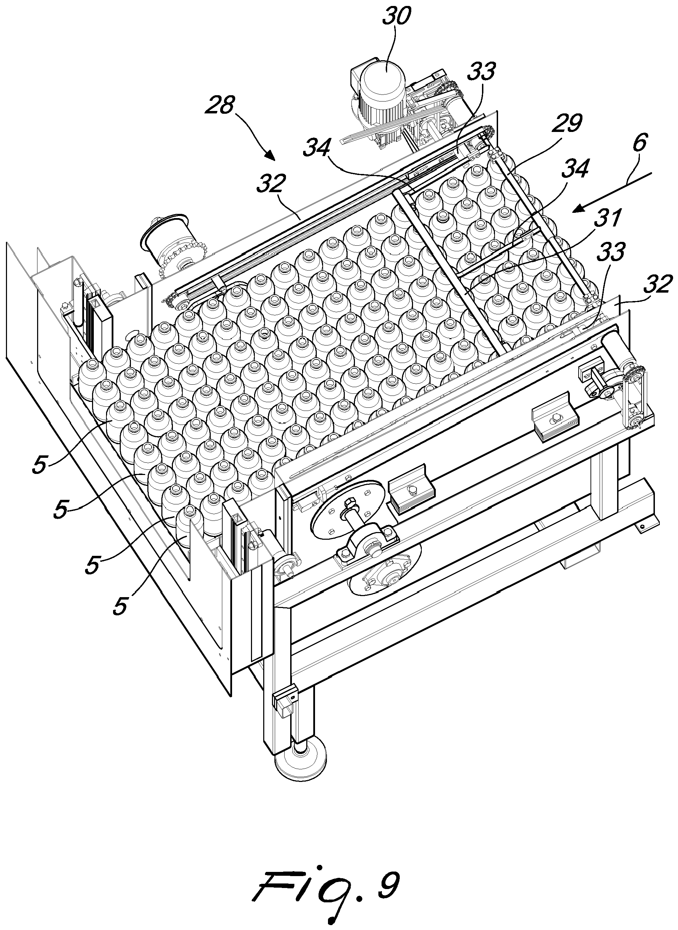

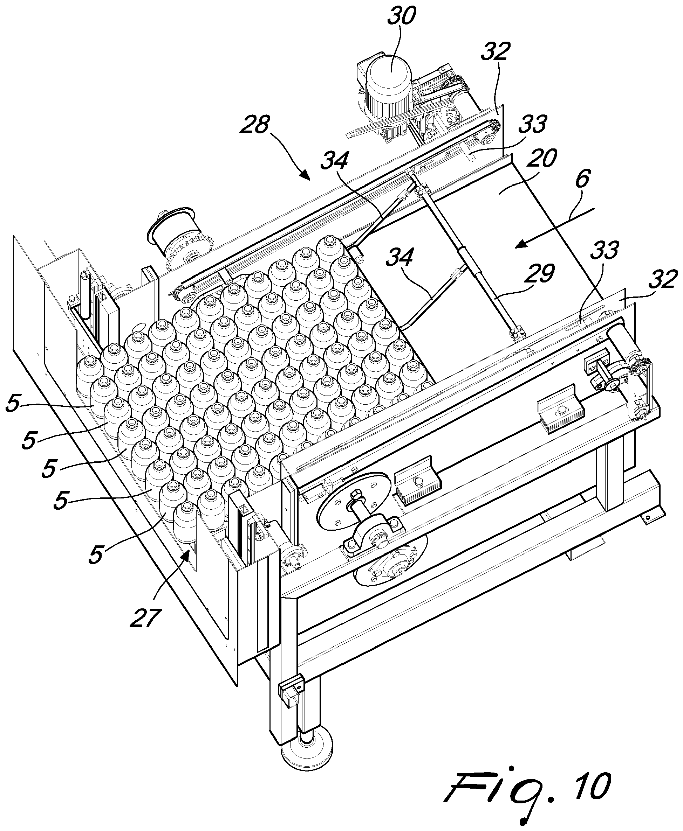

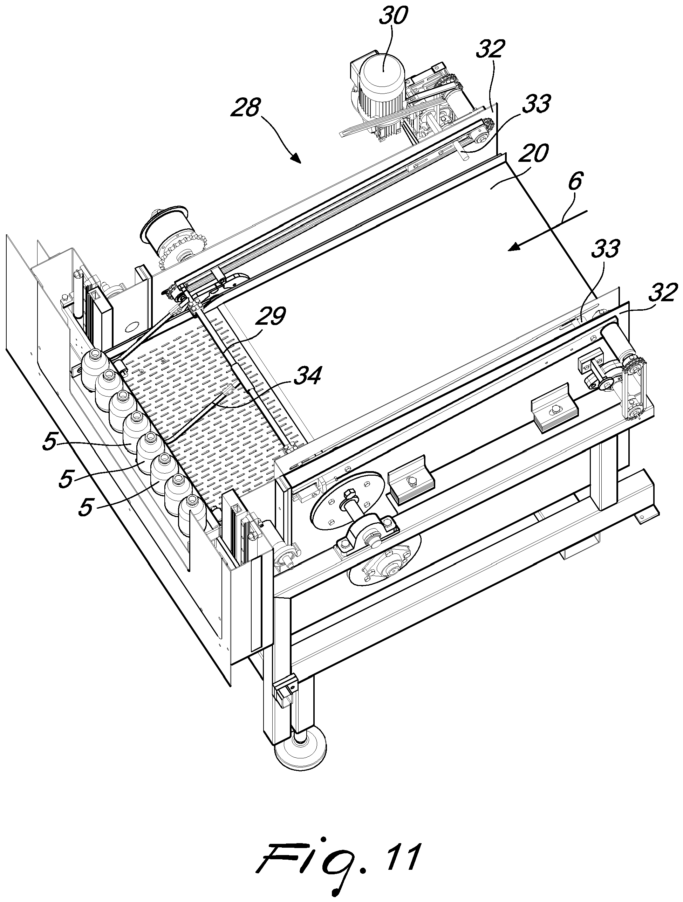

Advantageously, with particular reference to Figures 9 to 11, means 28 are provided for the additional advancement of the objects 5 arranged on the advancement line 6 for at least one unmoved portion of said advancement line 6 that is defined at at least one pair of planar elements 22 and 23 and/or at said unloading plane 27.

In the proposed embodiment, these additional advancement means 28 are provided exclusively only at the unloading plane 27, without however excluding that they might be installed in other portions of the advancement line 6.

In greater detail, in the proposed embodiment, said additional advancement means 28 comprise an advancement bar 29, which is arranged in an upward region and transversely at least with respect to the end portion of the conveyor belt 20 so as to not interfere with the objects 5 that move thereon and is movable horizontally by virtue of motor means 30 in a manner which is parallel to the advancement direction of the objects 5 between an initial position, located at an intermediate portion of the conveyor belt 20, and a final position, located proximate to the end of said conveyor belt 20.

Said additional advancement means 28 comprise furthermore an expulsion bar 31 which is integral in horizontal translation with the advancement bar 29 and is articulated thereto for its movement between an inactive position, in which said expulsion bar 31 is arranged upward and transversely to said end portion of the conveyor belt 20 in such a manner as to not interfere with the objects 5 that move thereon, and an active position, in which said expulsion bar 31 is arranged at a distance from the conveyor belt 20 such as to engage and push the objects 5 at their resting base to the end of the unmoved portion of the advancement line 6, i.e., to the end of the unloading plane 27.

More specifically, the expulsion bar 31 can move from the inactive position to the active position by means of a cam profile that is associated therewith and is formed on the side walls 32 of the thermal chamber 4 in which it operates and comprises a pair of pins 33 which are associated oppositely with respect to each other with said side walls 32 and are adapted to support the arms 34, by means of which the expulsion bar 31 is articulated to the advancement bar 29, for a first advancement portion of the latter from the initial position to the final position.

In this manner, the expulsion bar 31 is moved from the inactive position to the active position by gravity once the advancement bar 29 has moved beyond said pins 33 during its movement from the initial position to the final position.

Vice versa, the expulsion bar 31 is moved from the active position to the inactive position following the engagement of said arms 34 on the part of the pins 33 during the movement of the advancement bar 29 from the final position to the initial position.

The operation of sterilization and depyrogenation machine 1 for containers for chemical-pharmaceutical use, particularly of the energy-saving type, according to the present invention is clear and intuitive from what has been described so far.

The objects 5 for chemical-pharmaceutical use, which as already mentioned can consist of containers such as ampoules and/or bottles made of glass, aluminum or stainless steel, which exit from a washing machine, are placed on the first conveyor belt 18 along the advancement line 6 at the inlet of the first thermal chamber 2.

In greater detail, as shown in the cited figures, these objects 5 are arranged in parallel rows which are oriented transversely with respect to said advancement line 6 in such a manner that the objects 5 of each row are in contact with the objects 5 of the next row.

As the loading of the objects 5 occurs, the first conveyor belt 18 advances and the first thermal chamber 2 performs its thermal treatment, with which, by means of the laminar flow 7 of sterile filtered air in overpressure and preheated with the use of the air expelled to cool the treated objects 5, insulates the objects 5 that arrive from the washing machine against environmental particle contamination.

It is appropriate to point out that the first conveyor belt 18, being responsible for the conveyance of the objects 5 only and exclusively through the first thermal chamber 2, undergoes exclusively the thermal influence of said first thermal chamber 2, operating substantially at a constant temperature during the normal operation of the machine 1.

Once arrived at the end of the first conveyor belt 18, each row of objects 5 is pushed by the next row in such a manner as to slide on the coplanar connection means 21 formed by the two planar elements 22 and 23 interposed in mutual sequence between the conveyor belts 18 and 19.

As already mentioned, the particular comb-like shape of said two planar elements 22 and 23, their mutual sliding association by shape mating with play, their partial overlap with the adjacent conveyor belts 18 and 19, and the fact that the first planar element 22 forms a sliding plane that is parallel and arranged at a level that is higher than or equal to that of the sliding plane defined by the second planar element 23, allow the correct transition of the objects 5 from the first thermal chamber 2 to the second thermal chamber 3, preventing any tipping of the objects 5 and compensating any different thermal expansions between the two planar elements 22 and 23 as a consequence of the differences in operating temperature of the two thermal chambers 2 and 3.

Then, the objects 5 that exit from the first thermal chamber 2 are unloaded onto the second conveyor belt 19 at the inlet of the second thermal chamber 3, maintaining their orientation in parallel rows which are oriented transversely with respect to said advancement line 6 in such a manner that the objects 5 of each row are in contact with the objects 5 of the subsequent row.

As the loading of the objects 5 on the second conveyor belt 19 occurs, said belt advances and the second thermal chamber 3 performs its thermal treatment, by means of which, with a laminar flow 8 of air that is filtered and heated with electric resistance heaters 10, striking the objects 5 vertically downward from above, it progressively transfers heat to said objects 5, in order to heat them until they are fully sterilized.

It is appropriate to point out that the second conveyor belt 19, being responsible for the conveyance of the objects 5 only and exclusively through the second thermal chamber 3, undergoes exclusively the thermal influence of said second thermal chamber 3, operating substantially at a constant temperature during the normal operation of the machine 1.

In this manner, the objects 5 undergo heating from the bottom upward, by radiation and conduction, by virtue of the transfer of heat from the second conveyor belt 19 to said objects 5 which are colder and lie above it, adding to the heating by convection due to the laminar flow 8, which operates downward from above, thus allowing the second thermal chamber 3 to operate at a lower operating temperature than sterilization and depyrogenation machines of the known type.

Having arrived at the end of the second conveyor belt 19, each row of objects 5 is pushed by the next row in such a manner as to slide on the coplanar connection means 21 formed by the two planar elements 22 and 23 which are interposed in mutual sequence between the conveyor belts 19 and 20.

As already mentioned, the particular comb-like shape of said two planar elements 22 and 23, their mutual sliding association by shape mating with play, their partial overlap with the adjacent conveyor belts 19 and 20, and the fact that the first planar element 22 forms a sliding plane that is parallel and arranged at a level that is higher than or equal to that of the sliding surface defined by the second planar element 23, allow the correct transition of the objects 5 from the second thermal chamber 3 to the third thermal chamber 4, preventing any tipping of the objects 5 and compensating any different thermal expansions between the two planar elements 22 and 23 as a consequence of the differences in operating temperature of the two thermal chambers 3 and 4.

Subsequently, the objects 5 that exit from the second thermal chamber 3 are unloaded onto the third conveyor belt 20 at the inlet of the third thermal chamber 4, maintaining their orientation in parallel rows which are oriented transversely with respect to said advancement line 6 so that the objects 5 of each row are in contact with the objects 5 of the next row.

As the loading of the objects 5 onto the third conveyor belt 20 occurs, said belt advances and the third thermal chamber 4 performs its thermal treatment, by means of which, with a laminar flow 11 of filtered and cold air which strikes vertically downward from above, the objects 5 are cooled in such a manner that they are ready and suitable for the subsequent filling and closing steps.

It is appropriate to point out that the third conveyor belt 20, being responsible for the conveyance of the objects 5 only and exclusively through the third thermal chamber 4, also undergoes exclusively the thermal influence of said third thermal chamber 4, operating substantially at a constant temperature during the normal operation of the machine 1.

Having arrived at the end of the third conveyor belt 20, each row of objects 5 is pushed by the next row in such a manner as to slide on the unloading plane 27 arranged at the end of the last conveyor belt 20 along the advancement direction of the advancement line 6.

As already mentioned, by virtue of the particular shape of said unloading surface 27 and of its partial overlap with the third and last conveyor belt 20, the correct evacuation of the objects 5 from the third thermal chamber 4 is possible, preventing any tipping of the objects 5.

If the loading of objects 5 is interrupted, for example due to a change of format or a machine downtime, and therefore in the absence of a continuous thrust of the rows of subsequent objects 5 with respect to the preceding objects 5, the additional advancement means 28 intervene in order to allow the complete advancement of the objects 5 on the unloading plane 27.

In greater detail, as soon as the last row of objects 5 moves beyond the expulsion bar 31, which as described previously is arranged initially in its inactive position, i.e., arranged above and transversely to the conveyor belt 20 so as to not interfere with the objects 5 that are moving thereon, the motor means 30 actuate the advancement bar 29 from its initial position to its final position, consequently moving by gravity in a controlled manner, by virtue of the work performed by the pins 33 with the arms 34, the expulsion bar 31 from its inactive position to its active position, so as to push the objects 5 and clear completely the advancement line 6.

As soon as the advancement bar 29 reaches the stroke limit, it returns to the initial position, moving with it the expulsion bar 31, which also returns to its inactive position.

In practice it has been found that the sterilization and depyrogenation machine for containers for chemical-pharmaceutical use, particularly of the energy-saving type, according to the invention achieves fully the intended aim and objects.

With respect to machines of the known type which are provided with a single conveyor belt which warms up and cools continuously at each cycle, there is a considerable energy saving, since in the operating condition the energy for heating and cooling the individual conveyor belts, the maximum value in the energy calculation, must be considered nil by virtue of the splitting of the conveyance into individual sectors which are thermally independent and are equal in number to at least one for each individual thermal chamber.

In fact, each conveyor belt does not absorb energy, neither to warm up nor to cool down, since it operates at a substantially constant operating temperature which is equal to the operating temperature of the respective thermal chamber with which it is associated.

In this manner, it is possible to eliminate in the operating condition the energy consumption for the heating and cooling of the conveyor belt, entailing an energy saving comprised between 40% and 50% with respect to the power required by the sterilization and depyrogenation process that is needed by machines of the known type with a single conveyor belt.

Another advantage of the machine according to the present invention resides in that as regards the second thermal chamber, termed depyrogenation chamber, the heat exchange for raising the objects to be treated to their intended temperature occurs with a flow of hot air downward from above, which adds to the radiation and conduction effect that the second conveyor belt entails on the objects carried by it.

In such conditions, the heating of the bottom of the object, often in the presence of a residue of washing water, is facilitated, accelerating the heating curve of the coldest point of the container (bottom of the container).

In other words, with the conveyor belt already warm, one obtains the advantage of supplying heat from the bottom simultaneously with the heat supplied by the air, reducing the time required for the evaporation of the residual water inside the containers. In this condition the objects reach the depyrogenation temperature in a shorter time.

Consequently, for an equal length of the second chamber, a longer depyrogenation time is available, allowing a reduction of the temperature of the air to values of approximately 280°C with respect to 350°C to obtain the same result with a machine of the known type, with an additional energy saving in thermal dispersions which are proportional to the thermal gradient.

The energy for performing the cooling of the objects also is lower than that required with machines of the known type, since the conveyor belt of the third chamber is at a lower temperature, which is equal to the temperature of said chamber and requires no further cooling.

A further advantage of the machine according to the present invention resides in that if there are hazardous packagings (antibiotic powders, cytotoxic substances, etc.) downstream of the machine which with the single conveyor belt might be transferred to the entry area of the machine, with consequent environmental contamination, this does not occur in the case of split belts.

In this case, in fact, the flow is blocked and in the case of passage of the contaminant, due to pressure oscillations, said contaminant is inactivated by the high heating temperature of the depyrogenation chamber.

Finally, a last advantage of the machine according to the present invention resides in that the containers are heated to lower temperatures with respect to machines of the known type, with a consequent greater machinability in the subsequent operations for filling, closing, etc.

The sterilization and depyrogenation machine for containers for chemical-pharmaceutical use, particularly of the energy-saving type, thus conceived is susceptible of numerous modifications and variations, all of which are within the scope of the appended claims.

All the details may furthermore be replaced with other technically equivalent elements.

In practice, the materials used, as well as the contingent shapes and dimensions, may be any according to the requirements and the state of the art.

The disclosures in Italian Patent Application no. 102016000115684 (UA2016A008276), from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A sterilization and depyrogenation machine (1) for containers for chemical-pharmaceutical use, particularly of the energy-saving type, comprising a plurality of thermal chambers (2, 3, 4) adapted to perform a process for the sterilization and depyrogenation of objects (5) for chemical-pharmaceutical use and arranged mutually in sequence along an advancement line (6) of said objects (5) in a thermally independent manner, said thermal chambers (2, 3, 4) being provided with openings for the entry and exit of said objects (5) which are formed transversely along and with respect to said advancement line (6) in such a manner as to give said machine (1) a tunnel-like configuration, **characterized in that** it comprises means (14) for the advancement of said objects (5) which are adapted to transfer said objects (5) through said thermal chambers (2, 3, 4) and are arranged mutually in sequence along said advancement line (6) so as to define a plurality of advancement sectors (15, 16, 17), which are equal in number to at least one for each one of said thermal chambers (2, 3, 4), so that each one of said advancement means (14) that belongs to the same one of said advancement sectors (15, 16, 17) undergoes the thermal influence of only one of said thermal chambers (2, 3, 4).

2. The machine (1) according to claim 1, **characterized in that** said advancement means (14) comprise a plurality of conveyor belts (18, 19, 20), one for each one of said advancement sectors (15, 16, 17), each one of said conveyor belts (18, 19) forming a horizontal advancement plane that is substantially coplanar to the horizontal advancement plane defined by the consecutive one of said conveyor belts (19, 20).

3. The machine (1) according to claim 2, **characterized in that** said conveyor belts (18, 19, 20) are motorized independently from and synchronously with one another.

4. The machine (1) according to claim 2 or 3, **characterized in that** said conveyor belts (18, 19, 20) have a continuous motion and are entirely accommodated inside the respective one of said thermal chambers (2, 3, 4) in such a manner as to keep the temperature of each one of said conveyor belts (18, 19, 20) substantially constant with said machine (1) in the operating condition.

5. The machine (1) according to one or more of the preceding claims, **characterized in that** it comprises coplanar connection means (21) which are formed between each pair of said conveyor belts (18, 19, 20) and are adapted to allow the passage by sliding of said objects (5) from each one of said conveyor belts (18, 19) to the next one.

6. The machine (1) according to claim 5, **characterized in that** for each pair of said conveyor belts (18, 19, 20), said coplanar connection means (21) comprise at least two planar elements (22, 23) which are interposed mutually in sequence between said conveyor belts (18, 19, 20) and are comb-shaped, so as to be mutually associated with the teeth of one accommodated with play in the recesses of the other, each one of said at least two planar elements (22, 23) being partially superimposed on the adjacent one of said conveyor belts (18, 19, 20) at the end part of said conveyor belt (18, 19, 20) with an edge (24) that is rounded on the opposite side with respect to the one designed to support said objects (5) in such a manner as to support said objects (5) as soon as said conveyor belt (18, 19) winds around its respective supporting roller.

7. The machine (1) according to claim 6, **characterized in that** said at least two planar elements (22, 23) are slidingly mutually associated along said advancement line (6) in such a manner as to compensate the thermal expansion of the material of which they are made.

8. The machine (1) according to claim 6 or 7, **characterized in that** the first one of each pair of said at least two planar elements (22), determined according to the direction of advancement of said advancement line (6), defines a sliding plane that is parallel and arranged at a level that is higher than or equal to that of the sliding plane formed by the second one of said at least two planar elements (23), determined according to the advancement direction of said advancement line (6), so as to prevent the teeth of said second planar element (23) from hindering the sliding of said objects (5) that arrive from said first planar element (22).

9. The machine (1) according to one or more of the preceding claims, **characterized in that** it comprises, for each pair of said thermal chambers (2, 3, 4), means (25) for compensating the thermal expansion of the warmest of said two thermal chambers (3) toward the coldest of said two thermal chambers (2, 4), so as to compensate the thermal expansion of the material of which said thermal chambers (2, 3, 4) are made, at the same time ensuring the insulation of said advancement line (6) from the outside environment both for the portions formed inside said thermal chambers (2, 3, 4) and for the transition portions formed between either of said thermal chambers (2, 3, 4).

10. The machine (1) according to claim 9, **characterized in that** said compensation means (25) comprise accordion structures (26) formed between said openings of two consecutive ones of said thermal chambers (2, 3,4).

11. The machine (1) according to one or more of the preceding claims, **characterized in that** it comprises an unloading plane (27) that is arranged at the end of the last one of said conveyor belts (20) along the advancement direction of said advancement line (6), said unloading plane (27) being partially superimposed on said last conveyor belt (20) at the end part of said last conveyor belt (20) with a rounded edge on the opposite side with respect to the one designed to support said objects (5) so as to support said objects (5) as soon as said last conveyor belt (20) winds onto its respective supporting roller.

12. The machine (1) according to one or more of the preceding claims, **characterized in that** it comprises means (28) for the additional advancement of said objects (5) arranged on said advancement line (6) for at least one portion that is not moved of said advancement line (6) formed at at least one pair of said at least two planar elements (22, 23) and/or at said unloading plane (27).

13. The machine (1) according to claim 12, **characterized in that** said additional advancement means (28) comprise an advancement bar (29), which is arranged above and transversely at least with respect to the end portion of the respective one of said conveyor belts (20) so as to not interfere with said objects (5) in motion thereon and is movable horizontally by virtue of motor means (30) in a manner that is parallel to the advancement direction of said objects (5) between an initial position, located at an intermediate portion of said conveyor belt (20), and a final portion, arranged proximate to the end of said conveyor belt (20), and an expulsion bar (31) that is integral in horizontal translation and is articulated to said advancement bar (29) for its movement between an inactive position, in which said expulsion bar (31) is arranged above and transversely to said end portion of said conveyor belt (20), so as to not interfere with said objects (5) that move thereon, and an active position, in which said expulsion bar (31) is arranged at a distance from said conveyor belt (20) such as to engage and push said objects (5) at their resting base up to the end of said at least one unmoved portion of said advancement line (6), said expulsion bar (31) being movable from said inactive position to said active position by means of a cam profile that is associated therewith and is formed on the side walls (32) of said thermal chamber (4) in which it operates.

14. The machine (1) according to claim 13, **characterized in that** said cam profile associated with said expulsion bar (31) comprises a pair of pins (33), associated in a mutually opposite manner to said side walls (32) and adapted to support the arms (34), by means of which said expulsion bar (31) is articulated to said advancement bar (29), for a first advancement portion of said advancement bar (29) from said initial position to said final position, said expulsion bar (31) moving from said inactive position to said active position by gravity once said advancement bar (29) has moved beyond said pins (33) during its movement from said initial position to said final position, said expulsion bar (31) moving from said active position to said inactive position following the engagement of said arms (34) by said pins (33) during the movement of said advancement bar (29) from said final position to said initial position.
